# FASCICULE DE BREVET EUROPEEN

(11) **EP 2 750 755 B1**
(45) Date de publication et mention de la délivrance du brevet: **01.04.2020**
(21) Numéro de dépôt: 12755839.3
(22) Date de dépôt: 30.08.2012
(51) Int. Cl.: A61B 17/12, A61M 5/142, A61M 39/02, A61M 39/04

(54) **NÉCESSAIRE DE MANOEUVRE D'UN ÉLÉMENT PRÉSENT DANS LE CORPS D'UN PATIENT, COMPRENANT UNE CHAMBRE IMPLANTABLE**
KIT ZUM MANÖVRIEREN EINES ELEMENTS IM KÖRPER EINES PATIENTEN MIT EINER IMPLANTIERBAREN KAMMER
KIT FOR MANOEUVRING AN ELEMENT PRESENT IN THE BODY OF A PATIENT, COMPRISING AN IMPLANTABLE CHAMBER

(30) Priorité: 02.09.2011 FR 1157814
(43) Date de publication de la demande: 09.07.2014
(73) Titulaire: Perouse Medical, 60173 Ivry le Temple (FR)
(72) Inventeur: PEROUSE, Eric, 75016 Paris (FR)
(74) Mandataire: Lavoix
(86) Numéro de dépôt international: PCT/EP2012/066911
(87) Numéro de publication internationale: WO 2013/030305

(56) Documents cités:
- WO-A1-2007/051563
- WO-A2-2007/138590
- US-A- 5 158 547

## Description

La présente invention concerne une chambre implantable destinée à être placée sous la peau d'un patient, selon le préambule de la revendication 1.

Une telle chambre implantable est utilisée pour fournir un point d'accès privilégié dans le corps d'un patient, lorsque des manipulations fréquentes doivent être effectuées, pour injecter un fluide dans le patient. Toutefois, les chambres implantables servent uniquement à cette injection et présentent donc des fonctionnalités limitées.

Les documents WO 2007/051563 A1, WO 2007/138590 A2, US 5 158 547 A décrivent des dispositifs implantés munis d'un élément mobile d'injection.

Par ailleurs, des dispositifs médicaux existent pour ligaturer un organe ou un vaisseau, par exemple dans le domaine de la chirurgie cardio-vasculaire, ou pour actionner à distance un dispositif implanté dans le corps d'un patient, par exemple une pompe.

De tels dispositifs nécessitent toutefois un accès direct à l'organe à ligaturer ou à l'objet à actionner, en ouvrant la peau du patient. Ainsi, si un praticien doit régulièrement serrer, desserrer ou actionner un même objet externe, il peut être amené à ouvrir et suturer plusieurs fois le corps du patient à l'endroit où de telles interventions sont pratiquées. Ceci peut être très contraignant, à la fois pour le patient et pour le praticien.

Un but de l'invention est d'obtenir une chambre implantable présentant des fonctionnalités améliorées.

A cet effet, l'invention a pour objet une chambre implantable selon la revendication 1.

Suivant d'autres modes de réalisation, la chambre implantable comprend une ou plusieurs des caractéristiques des revendications 1 à 10 ou des caractéristiques suivantes, prises isolément ou suivant toutes les combinaisons techniquement possibles :
- l'axe de rotation de l'organe rotatif est confondu avec l'axe de centrage de l'organe de centrage,
- l'organe de déplacement est propre à actionner ou à mettre en mouvement un élément situé dans le corps du patient, tel qu'un objet externe ou un organe, sans injecter de fluide hors du corps de base.

L'invention a en outre pour objet un nécessaire de manœuvre à distance d'un élément présent dans le corps d'un patient selon la revendication 11.

L'invention a également pour objet un procédé de manœuvre à distance d'un élément présent dans le corps d'un patient, le procédé comprenant les étapes suivantes :
- fourniture d'un nécessaire tel que défini plus haut,
- piquage de l'outil à travers le septum pour introduire la région distale de l'outil dans le volume intérieur,
- engagement de l'outil sur le mécanisme d'entraînement,
- actionnement du mécanisme d'entraînement à l'aide de l'outil pour déplacer l'organe de déplacement.

L'invention sera mieux comprise à la lecture de la description qui va suivre, donnée uniquement à titre d'exemple et faite en se référant aux dessins annexés, sur lesquels :
- la figure 1 est une vue schématique d'un premier nécessaire selon l'invention pour l'actionnement à distance d'un objet externe dans le corps d'un patient, comportant un outil d'actionnement et une chambre implantable ;
- la figure 2 est une vue en perspective extérieure de la chambre implantable de la figure 1 ;
- la figure 3 est une vue, prise en coupe selon un plan vertical médian de la chambre de la figure 1, comportant un mécanisme d'entraînement et une chaîne dentée ;
- la figure 4 est une vue, prise en coupe suivant un plan vertical médian du nécessaire de la figure 1 ;
- la figure 5 est une vue en perspective extérieure du mécanisme d'entraînement de la chambre de la figure 1, selon un premier mode de réalisation ;
- la figure 6 est une vue de dessus du mécanisme d'entraînement de la chambre de la figure 1, selon une variante de réalisation du mécanisme d'entraînement; et
- les figures 7, 8 et 9 sont des vues analogues à la figure 3 d'un deuxième nécessaire selon l'invention.

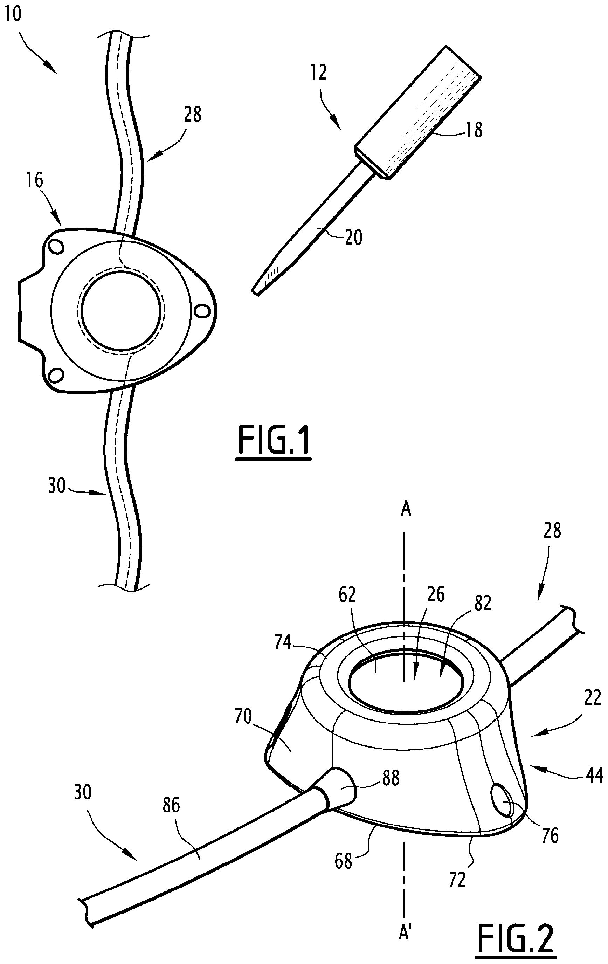

Un premier nécessaire 10 d'actionnement à distance d'un objet externe dans le corps d'un patient 11 est représenté sur la figure 1. Un tel nécessaire 10 est notamment propre à actionner à distance un élément situé dans le corps du patient 11, tel qu'un objet externe ou un organe.

Le nécessaire 10 comporte un outil 12 destiné à être inséré à travers la peau 14 du patient 11 et à actionner un mécanisme d'entrainement, et une première chambre implantable 16 selon l'invention, disposée sous la peau 14 du patient.

L'objet externe est par exemple une pompe médicale 17 située dans le corps du patient.

L'outil 12 comporte une région proximale 18 de manœuvre propre à être saisie par un utilisateur et une région distale 20 propre à percer un septum de la chambre 16 et à actionner un mécanisme d'entrainement.

Comme illustré par les figures 2 à 4, la chambre 16 comporte un corps de base 22 délimitant un volume intérieur 24, un septum 26 obturant le volume intérieur 24 et destiné à être piqué par la région 20 de l'outil 12. Elle définit au moins un conduit de passage 28, 30 faisant saillie à l'écart du corps de base 22. Le conduit de passage 28, 30 est rigide et solidaire du corps de base 22.

Dans cet exemple, la chambre 16 définit un premier conduit de passage 28 et un deuxième conduit de passage 30.

La chambre 16 comporte en outre des moyens de guidage de l'outil formés dans cet exemple par un cône de centrage 32 monté sous le septum 26 au sein du volume intérieur 24 du corps de base 22.

La chambre 16 comporte en outre un organe mobile de déplacement destiné à actionner l'objet extérieur, formé dans cet exemple par une chaîne dentée 36 apte à faire saillie hors du corps de base 22 à travers les conduits de passage 28, 30 et un mécanisme d'entraînement 38 de l'organe de déplacement, le mécanisme étant reçu dans le volume intérieur 24 du corps de base 22.

La chambre 16 comporte en outre des moyens libérables de blocage du mécanisme d'entraînement 38. Ces moyens de blocage comportent ici un anneau cranté 39 de retenue du mécanisme d'entraînement 38 monté sous le cône de centrage 32 au sein du volume intérieur 24 du corps de base 22, et un ressort 40 de sollicitation situé sous le mécanisme d'entraînement 38 au sein du volume intérieur 24 du corps de base 22.

Le corps de base 22 comporte un récipient intérieur 42 d'un axe central A-A' et une coque périphérique extérieure 44 qui, dans cet exemple, est encliquetée sur le récipient intérieur 42.

Dans cet exemple, le récipient 42 comprend une cuve métallique 46, un insert inférieur 48 monté autour de la cuve 46 et une bague 50 de retenue du septum 26 disposée au-dessus de la cuve 46. En variante, la cuve 46, l'insert 48 et la bague 50 sont réalisés d'une seule pièce.

La cuve 46 est par exemple formée en titane. Elle comporte un fond 52 qui délimite le fond du volume intérieur 24 et partiellement celui de la chambre 16 et une paroi latérale 54 sensiblement cylindrique qui délimite latéralement le volume intérieur 24.

La paroi latérale 54 présente un épaulement annulaire 56 d'appui du septum 26 qui s'étend le long de son bord supérieur. Cet épaulement définit une surface inférieure 58 de serrage du septum 26.

L'insert 48 s'étend autour de la paroi latérale 54 de la cuve, sous l'épaulement annulaire 56.

La bague 50 s'étend en regard de l'épaulement annulaire 56 et définit une surface supérieure 60 de serrage du septum 26.

Le volume intérieur 24 est délimité entre la paroi latérale 54 et le fond de la cuve 52. Le volume 24 s'ouvre vers le haut par une ouverture supérieure principale 62 recevant le septum 26. Il s'ouvre latéralement à travers la paroi latérale 54 de la cuve par une première ouverture radiale 64 et une deuxième ouverture radiale 66 de passage de l'organe mobile de déplacement.

L'insert 48 délimite deux passages radiaux à travers lesquels s'étend l'organe mobile de déplacement.

La coque périphérique 44 est réalisée en matière plastique, par exemple en polyoxyméthylène ou POM. Elle s'étend autour de l'insert 48 et de la bague 50, pour maintenir en position la bague 50 et l'insert 48 sur la cuve 46.

La coque 44 présente une surface inférieure 68 qui affleure le fond 52 du récipient 42 et une surface supérieure concave 70 qui converge de haut en bas depuis la bague 50 vers un bord périphérique 72 de la coque 44 situé au niveau de la surface inférieure 68.

La coque 44 présente une lèvre périphérique 74 sensiblement parallèle à la surface inférieure 68 qui s'appuie sur la bague 50 autour de l'ouverture principale 62.

Le bord périphérique 72 délimite le contour extérieur de la chambre 16. Dans cet exemple, ce contour est de forme sensiblement triangulaire.

La coque périphérique 44 délimite en outre des ouvertures traversantes 76 de passage d'un fil de suture, une première ouverture radiale 78 et une deuxième ouverture radiale 80 de passage de l'organe mobile de déplacement.

L'ouverture radiale 78 débouche en regard de l'ouverture 64 qu'elle prolonge radialement par rapport à l'axe A-A'. L'ouverture radiale 80 débouche en regard de l'ouverture 66 qu'elle prolonge radialement par rapport à l'axe A-A'.

Le septum 26 comporte un bloc 82 de base, réalisé en un matériau étanche. Le septum 26 est par exemple du type décrit dans la demande FR 10 52532 de la Demanderesse.

Le bloc 82 présente un contour extérieur de forme sensiblement complémentaire au contour de l'ouverture supérieure 62.

Le bloc 82 est enserré à sa périphérie entre la bague 50 et l'épaulement 56.

Il obture vers le haut le volume intérieur 24 de manière étanche. Ainsi, il empêche les fluides corporels et les tissus de pénétrer dans le volume intérieur 24. Ceci protège le mécanisme d'entraînement 38 et permet son maintien dans le corps pendant une durée significative.

Le bloc 82 est en outre propre à être percé par l'outil 12 lorsque le mécanisme d'entraînement 38 et l'organe mobile de déplacement doivent être déplacés.

Le premier conduit de passage 28 reçoit l'organe mobile de déplacement.

Il fait saillie radialement par rapport au corps de base 22 à travers l'ouverture radiale 78 et au-delà de cette ouverture radiale 78. Il est avantageusement réalisé à base d'un conduit rigide.

Le deuxième conduit de passage 30 reçoit aussi l'organe mobile de déplacement. Il comprend un raccord rigide creux 84, une tubulure rigide 86 et une bague de sertissage 88 de la tubulure rigide 86 sur le raccord rigide 84.

Il fait saillie radialement par rapport au corps de base 22 à travers l'ouverture radiale 80 et au-delà de cette ouverture radiale 80.

La tubulure 86 est réalisée à base d'une matière plastique rigide. Elle présente une longueur supérieure à l'étendue transversale maximale du corps de base 22, par exemple au moins deux fois supérieure à cette étendue maximale.

La tubulure 86 s'étend entre une première extrémité 92 engagée en force autour de l'extrémité libre du raccord rigide 84 et une deuxième extrémité 94 destinée à être reliée à la pompe médicale 17, située dans le corps du patient 11. La deuxième extrémité 94 définit un point d'appui sur la pompe médicale 17.

Le cône de centrage 32 présente une surface intérieure 96 qui converge de haut en bas depuis le septum 26 vers le mécanisme d'entraînement 38.

Le cône de centrage 32 est propre à guider le déplacement de l'outil 12 vers un axe de centrage lorsque l'outil 12 a traversé le septum 26 de haut en bas, afin de centrer l'outil 12 sur le mécanisme d'entraînement 38. Il débouche vers le bas en regard du mécanisme 38. L'axe de centrage du cône de centrage 32 est dans cet exemple confondu avec l'axe A-A'.

En variante, le cône de centrage 32 est remplacé par un tube de centrage présentant une surface interne de section constante.

L'anneau cranté 39 comporte dans cet exemple une denture périphérique intérieure. Il est propre à venir en prise autour du mécanisme d'entraînement 38 pour empêcher son déplacement.

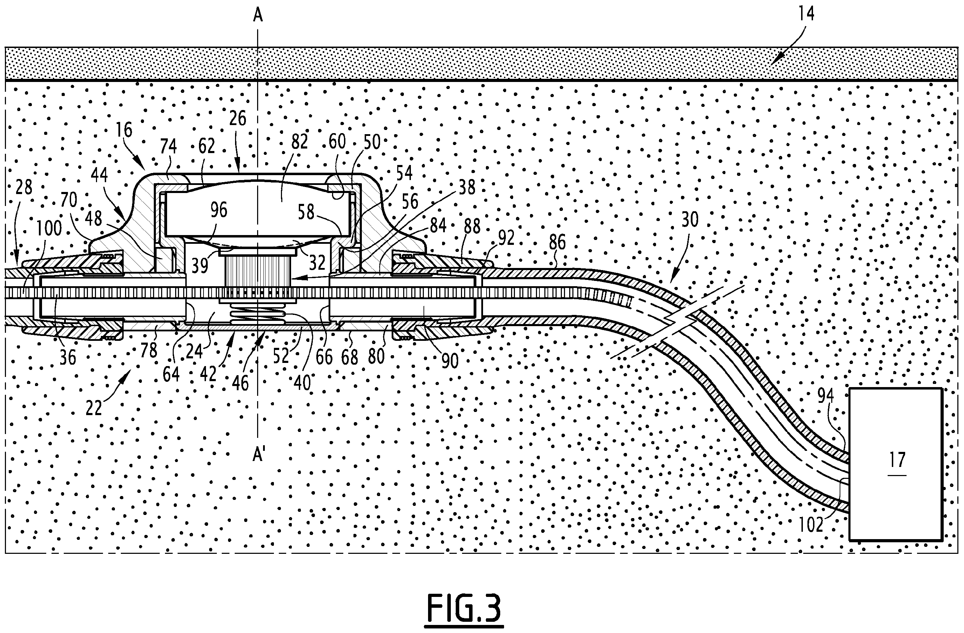

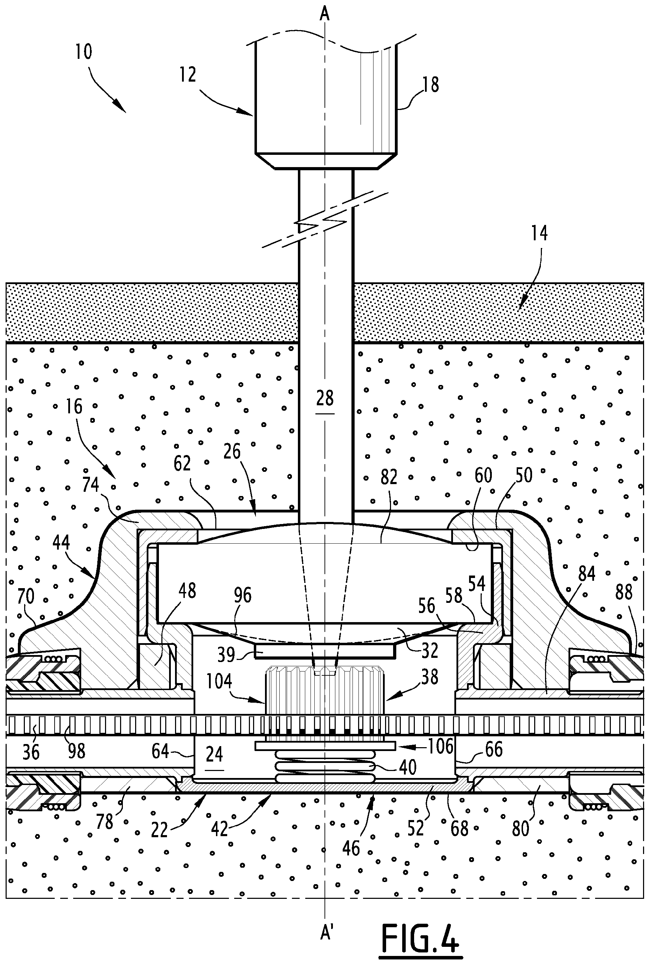

Dans l'exemple représenté sur les figures 3 et 4, l'anneau cranté 39 est avantageusement venu de matière avec le cône de centrage 32, qu'il prolonge vers le bas.

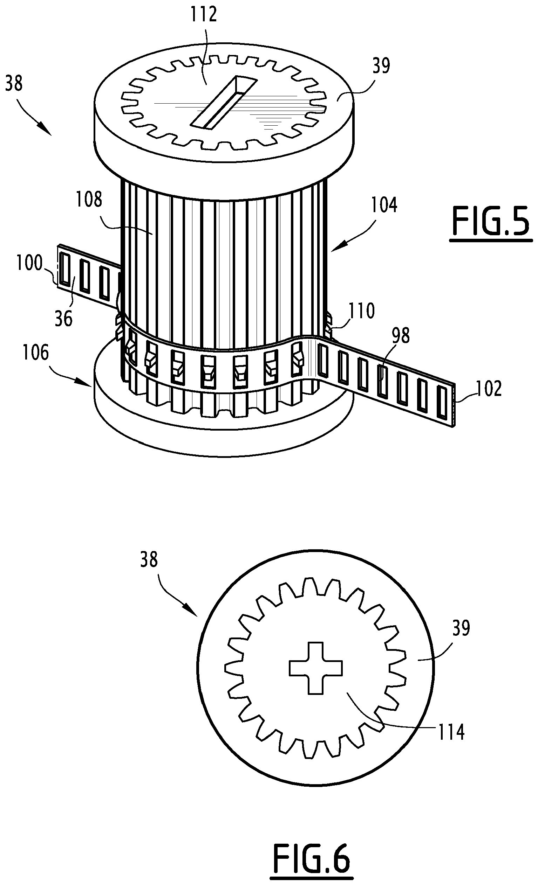

En référence à la figure 5, la chaîne dentée 36 comporte des orifices 98 propres à coopérer avec le mécanisme d'entraînement 38. Ces orifices sont formés par des fentes transversales espacées les unes des autres le long d'un axe de la chaîne.

La chaîne dentée 36 s'étend, à travers le premier conduit de passage 28, le volume intérieur 24 et le deuxième conduit de passage 30 respectivement, entre une première extrémité libre 100 et une deuxième extrémité libre 102 reliée à un actionneur de la pompe 17. L'extrémité libre 102 est apte à passer d'une position éloignée de la chambre 16 à une position rapprochée de la chambre 16 pour l'actionnement de la pompe 17 en étant entraînée par le mécanisme d'entraînement 38.

En variante, la chaîne dentée 36 est remplacée par une crémaillère, les orifices 98 étant remplacés par des crans propres à coopérer avec le mécanisme d'entraînement 38.

Le mécanisme d'entraînement 38 est propre à se déplacer longitudinalement selon l'axe A-A' entre une position bloquée engagée sur l'anneau 39 et une position libérée. Dans sa position libérée, il est apte en outre à déplacer la chaîne dentée 36 par rapport au corps de base 22.

Comme illustré sur les figures 4 et 5, le mécanisme d'entraînement 38 comporte un organe rotatif 104 et un support fixe 106 de guidage de l'organe rotatif.

L'organe 104 est monté rotatif dans le volume intérieur 24 de la chambre 16 autour de l'axe A-A'.

L'organe rotatif 104 comporte un cylindre rainuré 108 destiné à coopérer avec l'anneau 39, un pignon denté 110 pour l'entraînement de la chaîne 36, et une tête 112 munie d'un logement de réception de l'outil 12. Le cylindre rainuré 108, le pignon denté 110 et la tête 112 sont avantageusement venus de matière.

Le cylindre rainuré 108 est propre à s'insérer dans l'anneau cranté 39, les rainures extérieures du cylindre rainuré 108 étant parallèles à l'axe de rotation de l'organe rotatif 104.

Le pignon denté 110 est propre à s'enclencher dans les orifices 98 de la chaîne dentée 36.

La tête 112 est propre à coopérer avec la région distale 20 de l'outil 12.

L'axe de rotation de l'organe rotatif 104 est confondu avec l'axe de centrage A-A' du cône de centrage 32 de sorte que la région distale 20 est guidée vers le logement lors de l'insertion de l'outil 12 à travers le septum 26.

Dans cet exemple, le logement de réception présente une forme de fente droite.

En variante et comme illustré sur la figure 6, le logement dans la tête 114 présente une forme cruciforme.

Le support 106 est par exemple formé par un pion faisant saillie le long de l'axe A-A' dans le volume intérieur 24.

Le ressort 40 est interposé entre le fond de la cuve 46 et le mécanisme d'entraînement 38. Il engendre une force de sollicitation permanente du mécanisme d'entraînement vers sa position bloquée.

Lorsque le mécanisme d'entraînement 38 est dans sa position bloquée, le ressort 40 sollicite le mécanisme d'entraînement 38, maintenant ainsi l'organe rotatif 104 dans l'anneau cranté 39. L'organe rotatif 104 est alors bloqué en rotation autour de l'axe A-A'.

Lorsque le mécanisme d'entraînement 38 est dans sa position libérée, le ressort 40 sollicite le mécanisme d'entraînement vers sa position bloquée. L'organe rotatif 104 est dégagé de l'anneau cranté 39, et la rotation de l'organe rotatif 104 est alors possible.

Le fonctionnement du premier nécessaire 10 selon l'invention va maintenant être décrit.

Initialement, comme illustré par la figure 3, la chambre implantable 16 a été disposée chirurgicalement sous la peau 14 du patient 11. A cet effet, la chambre 16 est appliquée contre des tissus du patient et est maintenue en position en la suturant à travers les ouvertures traversantes 76. Le septum 26 est alors placé en regard de la peau 14, au voisinage de celle-ci pour recevoir facilement l'outil 12.

La tubulure rigide 86 est déployée dans le corps du patient pour relier l'extrémité 94 du conduit de passage 30 à la pompe implantable 17. A l'intérieur de la pompe 17, l'extrémité 102 de la chaîne dentée 36 est engagée avec un actionneur de la pompe 17.

Puis, la peau 14 du patient est remise en place, de sorte que la chambre 16 est partiellement ou totalement masquée par la peau 14.

Lorsque la pompe 17 doit être actionnée, par exemple pour la démarrer ou modifier son débit, le praticien effectue tout d'abord une radiographie du patient pour visualiser la position exacte de la chambre 16 et ses caractéristiques.

Ensuite, le praticien perce la peau 14 du patient avec la région distale 20 de l'outil 12. Il traverse ensuite le septum 26, en saisissant la région proximale 18 de manœuvre de l'outil 12 pour amener la région distale 20 de l'outil 12 dans le volume intérieur 24.

Le praticien déplace l'outil 12 le long de l'axe de centrage A-A' du cône de centrage 32, jusqu'à insérer la tête de l'outil 12 dans le logement de la tête 112 de l'organe rotatif 104.

Le praticien applique ensuite une force dirigée vers le fond de la cuve 46 le long de l'axe A-A' pour comprimer le ressort 40 et déplacer l'organe rotatif 104 vers sa position libérée. L'organe rotatif 104 est alors dégagé de l'anneau cranté 39.

Le praticien imprime ensuite un mouvement de rotation au mécanisme d'entraînement 38 via l'outil 12. La chaîne dentée 36 se déplace alors longitudinalement dans le conduit 30.

Ce déplacement commande la pompe 17, pour l'activer, la stopper, ou modifier son débit.

On conçoit ainsi que la pompe 17 est démarrée à distance par le praticien, et avec un risque minimal d'infection pour le patient. En effet, seule une incision à l'aide de l'outil 12 est nécessaire pour actionner la pompe 17.

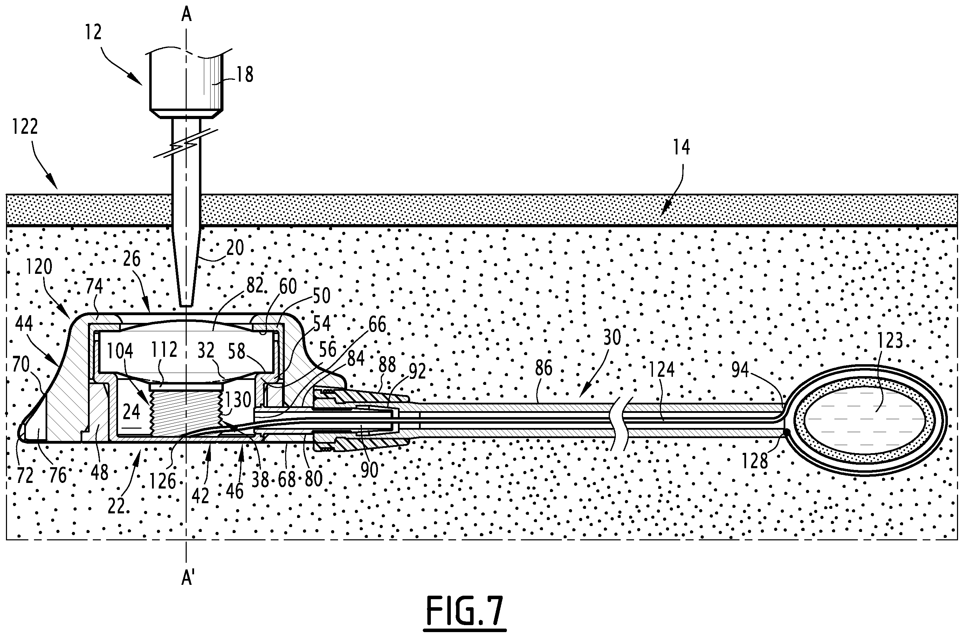

La chambre 120 d'un deuxième nécessaire 122 selon l'invention est représentée sur les figures 7, 8 et 9.

Une telle chambre 120 est notamment propre à ligaturer à distance un organe 123 ou un vaisseau sanguin du patient 11.

A la différence de la chambre 16 du premier nécessaire 10, la chambre 120 ne comporte qu'un seul conduit de passage 30 faisant saillie à l'écart du corps de base 22. Le conduit de passage 30 est rigide et solidaire du corps de base 22.

La tubulure 86 s'étend entre une première extrémité 92 engagée en force autour de l'extrémité libre du raccord rigide 84 et une deuxième extrémité libre 94 destinée à être placée à proximité immédiate de l'organe 123 à ligaturer.

Par ailleurs, l'organe mobile de déplacement est formé d'un fil 124, apte à faire saillie hors du corps de base 22 à travers le conduit de passage 30. Le fil 124 s'étend, à l'intérieur du volume intérieur 24 et du conduit de passage 30, entre une première extrémité 126 fixée à l'organe rotatif 104 et une deuxième extrémité 128 fixée sur l'extrémité libre 94 de la tubulure 86.

Le fil 124 définit à son extrémité distale une boucle de serrage de longueur active variable ceinturant l'organe 123.

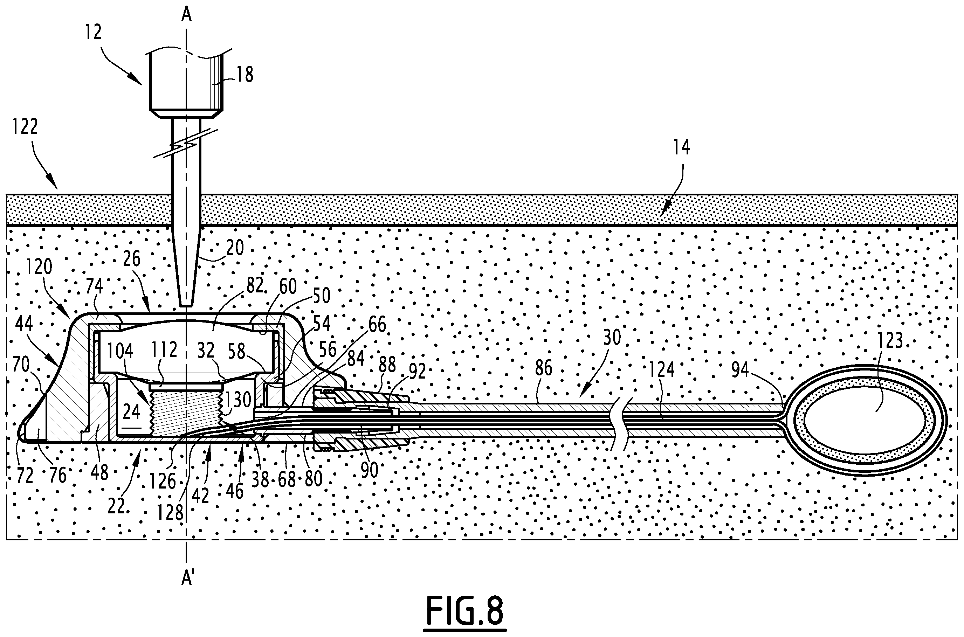

En variante, et comme illustré sur la figure 8, le fil 124 s'étend entre une première extrémité 126 et une deuxième extrémité 128, les deux extrémités 126, 128 étant fixées à l'organe rotatif 104. Le fil 124 définit une boucle de serrage de longueur active variable ceinturant l'organe 123 dans une région sensiblement proche du milieu du fil.

A la différence de la chambre 16 du premier nécessaire 10, la chambre 120 ne comporte pas d'anneau cranté, ni de ressort.

En outre, l'organe rotatif 104 comporte un cylindre fileté 130 et une tête 112 munie d'un logement de réception de l'outil 12. Le cylindre fileté 130 et la tête 112 sont avantageusement venus de matière. A la différence de la chambre 16 du premier nécessaire 10, l'organe rotatif 104 ne comporte pas de pignon denté.

Le filetage extérieur du cylindre 130 définit une rainure hélicoïdale d'enroulement de l'organe mobile de déplacement, destinée à recevoir le fil.

Initialement et comme illustré sur la figure 7, le fil 124 enserre l'organe 123 qui n'est pas comprimé.

Lorsque le praticien souhaite ligaturer localement l'organe 123, il effectue tout d'abord une radiographie du patient pour visualiser la position exacte de la chambre 120 et ses caractéristiques.

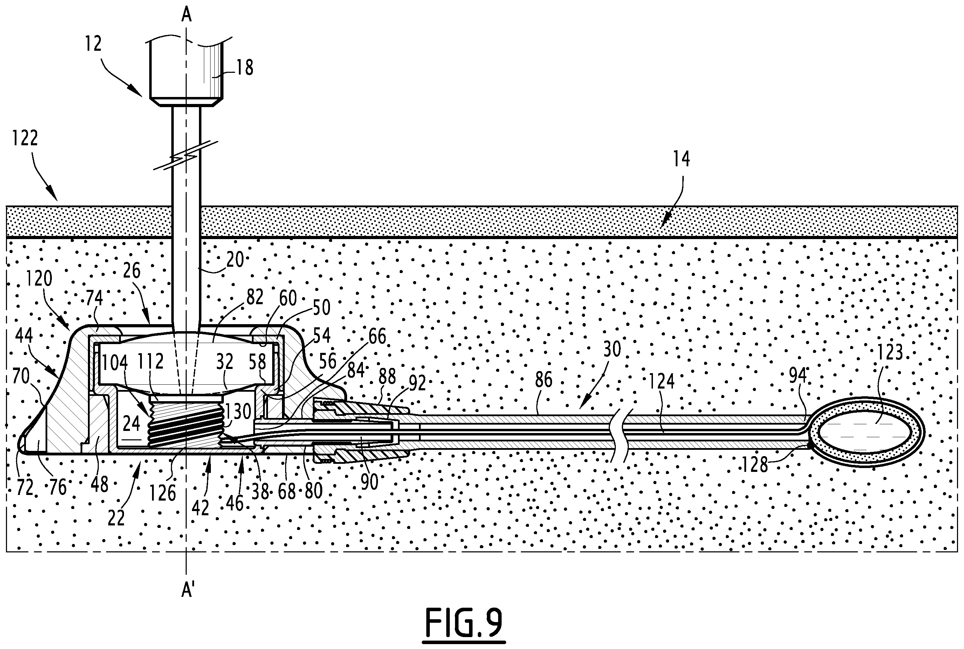

Comme illustré sur la figure 9, le praticien introduit l'outil 12 à travers la peau 14 du patient et traverse le septum 26, en saisissant la région proximale 18 de manœuvre de l'outil 12 pour amener la région distale 20 de l'outil 12 dans le volume intérieur 24.

Le praticien déplace alors l'outil 12 le long de l'axe de centrage du cône de centrage 32, jusqu'à insérer la tête de l'outil 12 dans le logement de la tête 112 de l'organe rotatif 104.

Le praticien imprime un mouvement de rotation au mécanisme d'entraînement 38 via l'outil 12, pour mettre en mouvement le fil 124. Ce mouvement enroule une longueur croissante de fil autour du cylindre 130, provoquant une diminution de la longueur du fil situé hors de la chambre 120.

En référence à la figure 9, la longueur active de la boucle de serrage diminue alors, provoquant l'enserrement local de l'organe 123.

A l'inverse, en entraînant en rotation le mécanisme d'entraînement 38 dans un sens opposé, le fil 124 se déroule hors du cylindre 130.

La longueur active de la boucle de serrage augmente alors, autorisant un déploiement de l'organe 123.

Grâce à l'invention qui vient d'être décrite, on conçoit que la chambre implantable selon l'invention présente des fonctionnalités améliorées, telles que par exemple l'actionnement à distance d'une pompe implantée au sein d'un patient, ou la ligature à distance d'un organe ou d'un vaisseau sanguin à l'aide d'un organe de déplacement actionné par un mécanisme reçu dans le volume intérieur de la chambre. La chambre implantable selon l'invention évite par ailleurs une colonisation cellulaire à l'intérieur du volume intérieur de la chambre, et protège ainsi le mécanisme d'entraînement. Elle limite enfin les risques d'infection pour le patient.

Comme il apparait clairement sur les figures, l'organe de déplacement 36, 124 est propre à actionner ou à mettre en mouvement un élément 17, 123 situé dans le corps du patient 11, sans avoir à injecter simultanément du fluide à travers l'organe de déplacement 36, 124.

En outre, la longueur active de l'organe de déplacement 36, 124 propre à être extraite hors du corps de base 22 dans le conduit de passage 28, 30 est supérieure à l'étendue transversale maximale du corps de base 22, notamment supérieure à deux fois l'étendue transversale du corps de base 22. Ceci permet d'actionner des éléments qui sont à distance de la chambre 16, 120, au delà du voisinage immédiat de la chambre 16, 120.

Qui plus est, l'organe de déplacement 36, 124 peut être extrait hors du conduit de passage 28, 30 sur une longueur significative, par exemple supérieure à 0,5 fois la longueur du conduit de passage 28, 30.

## Revendications

1. Chambre implantable (16 ; 120) destinée à être placée sous la peau (14) d'un patient (11), du type comprenant :
- un corps de base (22) creux délimitant un volume intérieur (24), le volume intérieur (24) débouchant par une ouverture principale (62),
- un septum (26) monté sur le corps de base (22) pour obturer l'ouverture principale (62),
- au moins un conduit de passage (28, 30) faisant saillie à l'écart du corps de base (22),
- un organe de déplacement (36 ; 124) mobile par rapport au corps de base (22), apte à faire saillie hors du corps de base (22) à travers le ou chaque conduit de passage (28, 30), et l'organe de déplacement étant propre à actionner ou à mettre en mouvement un objet externe (17) ou un organe (123) situé dans le corps du patient (11),
- un mécanisme d'entraînement (38) de l'organe de déplacement (36 ; 124) situé dans le volume intérieur (24) du corps de base (22) pour déplacer l'organe de déplacement (36 ; 124) par rapport au corps de base (22), et
**caractérisée en ce qu'**elle comporte :
- un organe de centrage (32) monté sous le septum (26) au sein du volume intérieur (24) du corps de base (22), l'organe de centrage (32) étant un cône de centrage ou tube de centrage, l'organe de centrage (32) étant propre à guider longitudinalement selon un axe de centrage le déplacement d'un outil (12),
le mécanisme d'entraînement (38) comportant un organe (104) monté rotatif dans le volume intérieur (24) autour d'un axe AA', l'organe de déplacement (36 ; 124) étant engagé sur l'organe rotatif (104), et l'axe AA' étant un axe de centrage d'un outil d'actionnement du mécanisme d'entraînement lors de son insertion à travers le septum (26),
le septum (26) étant destiné à être piqué par ledit outil (12) et le mécanisme d'entraînement (38) étant propre à coopérer avec ledit outil (12),
l'axe de rotation de l'organe rotatif (104) étant confondu avec l'axe de centrage AA' du cône de centrage (32) de sorte que la région distale est guidée vers le logement lors de l'insertion de l'outil (12) à travers le septum (26),
le cône de centrage (32) présentant une surface intérieure (96) convergeant de haut en bas depuis le septum (26) vers le mécanisme d'entrainement (38),
le cône de centrage (32) étant propre à guider le déplacement de l'outil (12) vers l'axe de centrage lorsque l'outil (12) a traversé le septum (26) de haut en bas, afin de centrer l'outil (12) sur le mécanisme d'entrainement (38),
le cône de centrage (32) débouchant vers le bas en regard du mécanisme (38).

2. Chambre implantable (16 ; 120) selon la revendication 1 **caractérisée en ce que** l'organe de centrage (32) comporte une surface intérieure convergente (96) vers une ouverture de centrage située en regard du mécanisme d'entraînement (38).

3. Chambre implantable (16 ; 120) selon l'une quelconque des revendications précédentes, **caractérisée en ce que** l'organe rotatif (104) comprend une tête (112, 114) délimitant un logement de réception d'un outil (12) d'actionnement du mécanisme d'entraînement (38).

4. Chambre implantable (16) selon l'une quelconque des revendications précédentes, **caractérisée en ce que** l'organe rotatif (104) présente un pignon denté (110), l'organe de déplacement (36) définissant des orifices (98) espacés suivant sa longueur pour coopérer avec le pignon denté (110) sur l'organe rotatif (104).

5. Chambre implantable (16) selon l'une quelconque des revendications précédentes, **caractérisée en ce que** l'organe de déplacement comprend des crans propres à coopérer avec l'organe rotatif (104).

6. Chambre implantable (16 ; 120) selon l'une quelconque des revendications précédentes **caractérisée en ce qu'**elle comporte des moyens de blocage (39, 40) du mécanisme d'entraînement situés au sein du volume intérieur (24) du corps de base (22), les moyens de blocage étant libérables lors de l'introduction d'un outil (12) d'actionnement du mécanisme d'entraînement (38).

7. Chambre implantable (16 ; 120) selon la revendication 6 **caractérisée en ce que** le mécanisme d'entraînement (38) comporte un organe (104) d'entraînement de l'organe de déplacement (36 ; 124), mobile entre une position bloquée et une position libérée, les moyens de blocage comportant :
- un organe (39) de mise en prise de l'organe (104) mobile dans la position bloquée, et
- un organe (40) de sollicitation élastique de l'organe (104) mobile vers sa position bloquée.

8. Chambre implantable (16; 120) selon l'une quelconque des revendications précédentes, **caractérisée en ce que** l'organe de déplacement (36 ; 124) est formé par un organe souple, notamment par une chaîne, une crémaillère, ou un fil.

9. Chambre implantable (16; 120) selon l'une quelconque des revendications précédentes, **caractérisée en ce que** le conduit de passage (28, 30) comporte une tubulure rigide (86) présentant une longueur supérieure à l'étendue transversale maximale du corps de base (22), notamment au moins deux fois supérieure à l'étendue transversale maximale du corps de base (22).

10. Nécessaire (10 ; 122) de manœuvre à distance d'un objet externe (17) ou d'un organe (123) présent dans le corps d'un patient (11), du type comprenant :
- une chambre implantable (16 ; 120) selon l'une quelconque des revendications précédentes, et
- un outil (12) comportant une région proximale (18) de manœuvre et une région distale (20) propre à percer le septum (26) pour actionner le mécanisme d'entraînement (38).

## Patentansprüche

1. Implantierbare Kammer (16; 120), die dazu bestimmt ist, unter der Haut (14) eines Patienten platziert zu werden, des Typs, aufweisend:
- einen hohlen Basiskörper (22), der ein Innenvolumen (24) begrenzt, wobei das Innenvolumen (24) über eine Hauptöffnung (62) ausmündet,
- eine Trennwand (26), die an dem Basiskörper (22) angebracht ist zum Verschließen der Hauptöffnung (62),
- wenigstens eine Durchgangsleitung (28, 30), die in Abstand von dem Basiskörper (22) vorsteht,
- ein Verlagerungsorgan (36; 124), das bezüglich des Basiskörpers (22) bewegbar ist und das geeignet ist, über den Basiskörper (22) hinaus vorzustehen durch die oder jede Durchgangsleitung (28, 30) hindurch, wobei das Verlagerungsorgan imstande ist, ein externes Objekt (17) oder ein Organ (123), das im Körper des Patienten (11) angeordnet ist, zu betätigen oder in Bewegung zu versetzen,
- einen Mechanismus zum Antreiben (38) des Verlagerungsorgans (36; 124), der im Innenvolumen (24) des Basiskörpers (22) angeordnet ist zum Verlagern des Verlagerungsorgans (36; 124) bezüglich des Basiskörpers (22),
**dadurch gekennzeichnet, dass** sie aufweist:
- ein Zentrierorgan (32), das unter der Trennwand (26) in dem Innenvolumen (24) des Basiskörpers (22) angebracht ist, wobei das Zentrierorgan (32) ein Zentrierkonus oder ein Zentrierrohr ist, wobei das Zentrierorgan (32) imstande ist, die Verlagerung eines Werkzeugs (12) entlang einer Zentralachse längs zu führen,
wobei der Mechanismus zum Antreiben (38) aufweist ein Organ (104), das in dem Innenvolumen (24) um eine Achse AA' drehbar angebracht ist, wobei das Verlagerungsorgan (36; 124) auf das drehbare Organ (104) gewickelt ist, und wobei die Achse AA' eine Zentrierachse eines Werkzeugs zur Betätigung des Mechanismus zum Antreiben ist während dessen Einsetzens durch die Trennwand (26) hindurch,
wobei die Trennwand (26) dazu bestimmt ist, von dem Werkzeug (12) durchstochen zu werden und der Mechanismus zum Antreiben (38) imstande ist, mit dem Werkzeug (12) zusammenzuwirken, wobei die Drehachse des drehbaren Organs (104) mit der Zentrierachse AA' des Zentrierkonus (32) zusammenfällt, sodass der distale Bereich zu der Aufnahme hin geführt wird während des Einsetzens des Werkzeugs (12) durch die Trennwand (26) hindurch,
wobei der Zentrierkonus (32) eine innere Fläche (96) hat, die von oben nach unten von der Trennwand (26) aus zu dem Mechanismus zum Antreiben (38) hin konvergiert,
wobei der Zentrierkonus (32) imstande ist, die Verlagerung des Werkzeugs (12) zu der Zentrierachse hin von oben nach unten zu führen, wenn das Werkzeug (12) die Trennwand (26) durchquert hat, um das Werkzeug (12) an dem Mechanismus zum Antreiben (38) zu zentrieren,
wobei der Zentrierkonus (32) nach unten hin gegenüber dem Mechanismus (38) ausmündet.

2. Implantierbare Kammer (16; 120) gemäß Anspruch 1, **dadurch gekennzeichnet, dass** das Zentrierorgan (32) eine innere Fläche (96) aufweist, die zu einer Zentrieröffnung hin konvergiert, welche gegenüber dem Mechanismus zum Antreiben (38) angeordnet ist.

3. Implantierbare Kammer (16; 120) gemäß irgendeinem der vorhergehenden Ansprüche, **dadurch gekennzeichnet, dass** das drehbare Organ (104) einen Kopf (112, 114) aufweist, der eine Aufnahme zum Aufnehmen eines Werkzeugs (12) zur Betätigung des Mechanismus zum Antreiben (38) aufweist.

4. Implantierbare Kammer (16) gemäß irgendeinem der vorhergehenden Ansprüche, **dadurch gekennzeichnet, dass** das drehbare Organ (104) ein Zahnritzel (110) hat, wobei das Verlagerungsorgan (36) Öffnungen (98) definiert, die entlang seiner Länge im Abstand sind, zum Zusammenwirken mit dem Zahnritzel (110) an dem drehbaren Organ (104).

5. Implantierbare Kammer (16) gemäß irgendeinem der vorhergehenden Ansprüche, **dadurch gekennzeichnet, dass** das Verlagerungsorgan Zacken aufweist, die imstande sind, mit dem drehbaren Organ (104) zusammenzuwirken.

6. Implantierbare Kammer (16; 120) gemäß irgendeinem der vorhergehenden Ansprüche, **dadurch gekennzeichnet, dass** sie aufweist Mittel zum Sperren (39, 40) des Mechanismus zum Antreiben, die in dem Innenvolumen (24) des Basiskörpers (22) angeordnet sind, wobei die Mittel zum Sperren freigebbar sind während des Einführens eines Werkzeugs (12) zum Betätigen des Mechanismus zum Antreiben (38).

7. Implantierbare Kammer (16; 120) gemäß Anspruch 6, **dadurch gekennzeichnet, dass** der Mechanismus zum Antreiben (38) aufweist ein Organ (104) zum Antreiben des Verlagerungsorgans (36; 124), das bewegbar ist zwischen einer Sperrposition und einer Freigabeposition, wobei die Mittel zum Sperren aufweisen:
- ein Organ (39) zum in Eingriff-Stehen mit dem bewegbaren Organ (104) in dessen Sperrposition, und
- ein Organ (40) zum elastischen Vorspannen des bewegbaren Organs (104) zu dessen Sperrposition hin.

8. Implantierbare Kamer (16; 120) gemäß irgendeinem der vorhergehenden Ansprüche, **dadurch gekennzeichnet, dass** das Verlagerungsorgan (36; 124) von einem biegeweichen Organ gebildet ist, insbesondere von einer Kette, einer Zahnleiste oder einem Draht.

9. Implantierbare Kammer (16; 120) gemäß irgendeinem der vorhergehenden Ansprüche, **dadurch gekennzeichnet, dass** die Durchgangsleitung (28, 30) ein starres Rohr (86) aufweist, das eine Länge hat, die größer ist als die maximale Querausdehnung des Grundkörpers (22), insbesondere wenigstens zweimal größer ist als die maximale Querausdehnung des Grundkörpers (22).

10. Gerät (10; 122) zum Fernbetätigen eines externen Objekts (17) oder eines Organs (12), das im Körper eines Patienten (11) vorliegt, des Typs, aufweisend:
- eine implantierbare Kammer (16; 120) gemäß irgendeinem der vorhergehenden Ansprüche, und
- ein Werkzeug (12), das einen proximalen Bereich (18) zur Betätigung und einen distalen Bereich (20) aufweist, der imstande ist, die Trennwand (26) zu durchstechen zum Betätigen des Mechanismus zum Antreiben (38).

## Claims

1. An implantable chamber (16; 120) designed to be placed under the skin (14) of a patient (11), comprising:
- a hollow main body (22) delimiting an internal volume (24), the internal volume (24) opening outwards via a main opening (62),
- a septum (26) mounted on the main body (22) to close the main opening (62),
- at least one conduit (28, 30) projecting away from the main body (22),
- a displacement member (36; 124) that is movable relative to the main body (22), capable of projecting from the main body (22) via the or each conduit (28, 30) and the displacement member (36; 124) being capable of actuating or setting in motion an external object (17) or an organ (123) situated in the body of the patient (11),
- a drive mechanism (38) for driving the displacement member (36; 124) situated in the internal volume (24) of the main body (22), in order to move the displacement member (36; 124) with respect to the main body (22), and
**characterized in that** it includes :
- a centering member (32) mounted below the septum (26) within the internal volume (24) of the main body (22), the centering member (32) being a centering cone or centering tube, the centering member (32) being capable of guiding the movement of a tool (12) longitudinally along a centering axis,
the drive mechanism (38) including a member (104) rotatably mounted in the internal volume (24) around an axis AA', the displacement member (36; 124) being engaged on the rotating member (104), and the axis AA' being a centering axis of a tool for actuating the drive mechanism during its insertion through the septum (26),
the septum (26) being designed to be pierced by said tool (12) and the drive mechanism (38) being capable of cooperating with said tool (12),
the axis of rotation of the rotating member (104) being coincident with the centring axis A-A' of the centring cone (32) so that the distal region is guided to the housing when inserting the tool (12) through the septum (26),
the centering cone (32) having an inner surface (96) converging up and down from the septum (26) toward the drive mechanism (38),
the centering cone (32) being adapted to guide the movement of the tool (12) towards the centering axis when the tool (12) has passed through the septum (26) from top to bottom, in order to center the tool (12) on the drive mechanism (38),
the centring cone (32) opening downwards opposite the mechanism (38).

2. The implantable chamber (16; 120) according to claim 1, **characterized in that** the centering member (32) includes an inner surface (96) converging toward a centering opening situated in register with the drive mechanism (38).

3. The implantable chamber (16; 120) according to any of the preceding claims, **characterized in that** the rotating member (104) comprises a head (112, 114) delimiting a housing for receiving a tool (12) for actuating the drive mechanism (38).

4. The implantable chamber (16) according to any of the preceding claims, **characterized in that** the rotating member (104) has a toothed pinion (110), the displacement member (36) defining orifices (98) spaced along its length to cooperate with the toothed pinion (110) on the rotating member (104).

5. The implantable chamber (16) according to any of the preceding claims, **characterized in that** the displacement member comprises notches capable of cooperating with the rotating member (104).

6. The implantable chamber (16 ; 120) according to any one of the preceding claims, **characterized in that** it includes means (39, 40) for locking the drive mechanism situated within the internal volume (24) of the main body (22), the locking means being able to be released when the actuating tool (12) for actuating the drive mechanism (38) is inserted.

7. The implantable chamber (16; 120) according to claim 6, **characterized in that** the drive mechanism (38) includes a drive member (104) for driving the displacement member (36; 124), movable between a locked position and a released position, the locking means including:
- a member (39) for engaging the displacement member (104) in the locked position, and
- an elastic bias member (40) for biasing the moving member (104) toward its locked position;

8. The implantable chamber (16; 120) according to any one of the preceding claims, **characterized in that** the displacement member (36; 124) is made up of a flexible member, in particular a chain, a rack or a thread.

9. An implantable chamber (16; 120), **characterized in that** the conduit (28, 30) includes a rigid tubing (86) having a length greater than the maximum transverse expanse of the main body (22), in particular at least two times greater than that maximum expanse of the main body (22).

10. A kit (10; 122) for remotely maneuvering an external object (17) or an organ (123) present in the body of a patient (11), of the type comprising:
- an implantable chamber (16; 120) according to any one of the preceding claims, and
- a tool (12) including a proximal maneuvering region (18) and a distal region (20) capable of piercing the septum (26) to actuate the drive mechanism (38).
